# EUROPEAN PATENT SPECIFICATION

(11) **EP 3 349 710 B1**
(45) Date of publication and mention of the grant of the patent: **02.07.2025**
(21) Application number: 16763860.0
(22) Date of filing: 13.09.2016
(51) Int. Cl.: A61G 11/00, A61B 5/097, G01N 33/497

(54) **NEONATAL INCUBATOR**
NEUGEBORENEN-INKUBATOR
INCUBATEUR NÉONATAL

(30) Priority: 15.09.2015 EP 15185215
(43) Date of publication of application: 25.07.2018
(73) Proprietor: Koninklijke Philips N.V., 5656 AG Eindhoven (NL)
(72) Inventor: MEFTAH, Mohammed, 5656 AE Eindhoven (NL); NIJSEN, Tamara, Mathea, Elisabeth, 5656 AE Eindhoven (NL)
(74) Representative: Philips Intellectual Property & Standards
(86) International application number: PCT/EP2016/071592
(87) International publication number: WO 2017/046103

(56) References cited:
- EP-A1- 2 762 880
- EP-A1- 2 762 880
- WO-A1-2012/064252
- WO-A1-2012/064252
- WO-A1-2014/076493
- WO-A1-2014/076493
- WO-A1-2014/076493
- WO-A1-2014/165732
- WO-A2-02/053079
- WO-A2-02/053079
- GB-A- 2 316 617
- GB-A- 2 316 617
- US-A1- 2009 104 074
- US-A1- 2009 104 074
- US-A1- 2009 308 136
- US-A1- 2015 126 804
- U COLARETA UGARTE ET AL: "Emission of volatile organic compounds from medical equipment inside neonatal incubators", JOURNAL OF PERINATOLOGY, vol. 34, no. 8, 1 August 2014 (2014-08-01), GB, pages 624 - 628, XP055256403, ISSN: 0743-8346, DOI: 10.1038/jp.2014.65
- ROZE J C ET AL: "Measurement of oxygen uptake in newborn infants during assisted and spontaneous ventilation", RESPIRATION PHYSIOLOGY, AMSTERDAM, NL, vol. 98, no. 2, 1 October 1994 (1994-10-01), pages 227 - 239, XP025197688, ISSN: 0034-5687, [retrieved on 19941001], DOI: 10.1016/0034-5687(94)00056-5
- U COLARETA UGARTE ET AL: "Emission of volatile organic compounds from medical equipment inside neonatal incubators", JOURNAL OF PERINATOLOGY, vol. 34, no. 8, 1 August 2014 (2014-08-01), GB, pages 624 - 628, XP055256403, ISSN: 0743-8346, DOI: 10.1038/jp.2014.65
- ROZE J C ET AL: "Measurement of oxygen uptake in newborn infants during assisted and spontaneous ventilation", RESPIRATION PHYSIOLOGY, AMSTERDAM, NL, vol. 98, no. 2, 1 October 1994 (1994-10-01), pages 227 - 239, XP025197688, ISSN: 0034-5687, [retrieved on 19941001], DOI: 10.1016/0034-5687(94)00056-5
- PACCAUD C ET AL: "Hand-disinfectant alcoholic vapors in incubators", JOURNAL OF NEONATAL PERINATAL MEDICINE, IOS PRESS, NL, vol. 4, 1 January 2011 (2011-01-01), pages 15 - 19, XP008179346, ISSN: 1934-5798, DOI: 10.3233/NPM-2011-2727
- P PRAZAD ET AL: "Airborne concentrations of volatile organic compounds in neonatal incubators", JOURNAL OF PERINATOLOGY, vol. 28, no. 8, 19 June 2008 (2008-06-19), GB, pages 534 - 540, XP055256397, ISSN: 0743-8346, DOI: 10.1038/jp.2008.75
- U COLARETA UGARTE ET AL: "Emission of volatile organic compounds from medical equipment inside neonatal incubators", JOURNAL OF PERINATOLOGY, vol. 34, no. 8, 24 April 2014 (2014-04-24), GB, pages 624 - 628, XP055256403, ISSN: 0743-8346, DOI: 10.1038/jp.2014.65

## Description

### TECHNICAL FIELD OF THE INVENTION

The present invention is directed generally to neonatal care. More particularly, an improved neonatal incubator is disclosed.

### BACKGROUND OF THE INVENTION

Physical contact with newborns residing in neonatal incubators, especially by persons other than the newborn's mother, should be avoided as much as possible, as such contact may cause the newborn stress and/or expose it to contaminants. Placing a newborn in an incubator may create an opportunity to employ minimally-invasive techniques, such as gas analysis. However, existing gas analysis techniques for diagnosing neonatal disease, while less invasive than other techniques (e.g., obtaining blood samples), still require physical contact with the newborn, e.g., to position a gas mask over the newborn's mouth and nose and/or to insert air tubes into the newborns nostrils. Thus, there is a need in the art to provide a less-invasive, and preferably non-invasive, way to perform gas analysis of a newborn's exhaled breath, as well as of the newborn's skin, feces and/or urine.

WO 2014/165732 discloses a system for monitoring an exhaled breath of a subject. WO 2014/076493 discloses a device for measurement of breath VOCs which uses a spectroscopy unit. US 2009/0308136 discloses gas chromatograph. "Hand-disinfectant alcoholic vapors in incubators" of C. Paccaud et. al., Journal of Neonatal Perinatal Medicine, vol 4, 1 Jan. 2011 pp. 15-19 (XP008179346) discloses analysis of gas in an incubator chamber to observe the effect of an inserted hand after hand sanitization. Analyzing the emission of VOCs from medical equipment inside a neonatal incubator is disclosed in Colareta Ugarte U, Prazad P, Puppala BL, Schweig L, Donovan R, Cortes DR, Gulati A. Emission of volatile organic compounds from medical equipment inside neonatal incubators. J Perinatol. 2014 Aug;34(8):624-8. doi: 10.1038/jp.2014.65. Epub 2014 Apr 24. PMID: 24762411. Document WO 02/053079 A2 discloses a neonatal incubator with a humidity sensor and oxygen cells. Document GB 2 316 617 A discloses a neonatal incubator with an alarming means responsive to the opening and closing of the doors of the incubator.

### SUMMARY

The invention is defined by the claims. The present disclosure is directed to methods and apparatuses for non-invasive neonatal gas analysis. For example, in various embodiments, a neonatal gas analysis apparatus may be configured to obtain one or more samples of gas from an interior of a neonatal incubator. These samples may contain various types of molecules of interest, such as biomolecules, carbohydrates, volatile organic compounds ("VOCs"), etc., that may be carried in an infant's breath, feces, skin, and/or urine. The neonatal gas analysis apparatus may isolate, capture, and/or concentrate one or more types of molecules from the one or more gas samples, and analyze the isolated, captured and/or concentrated biomolecules to determine one or more health indicators associated with an infant residing in the neonatal incubator.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically illustrates an example neonatal incubator equipped with a neonatal gas analysis system configured with selected aspects of the present disclosure, in accordance with various embodiments.
Fig. 2 schematically illustrates an example process for performing neonatal gas analysis using a neonatal incubator, in accordance with various embodiments.

### DETAILED DESCRIPTION OF THE EMBODIMENT

Physical contact with newborns residing in neonatal incubators, especially by persons other than the newborn's mother, should be avoided as much as possible, as such contact may cause the newborn stress. Existing techniques for diagnosing neonatal disease using gas analysis, while less invasive than other techniques, still require physical contact with the newborn. Accordingly, Applicants have recognized and appreciated that it would be beneficial to provide a less-invasive, and preferably non-invasive and unobtrusive, way to perform gas analysis of an incubated neonatal. In view of the foregoing, various embodiments and implementations of the present disclosure are directed to a neonatal gas analysis apparatus.

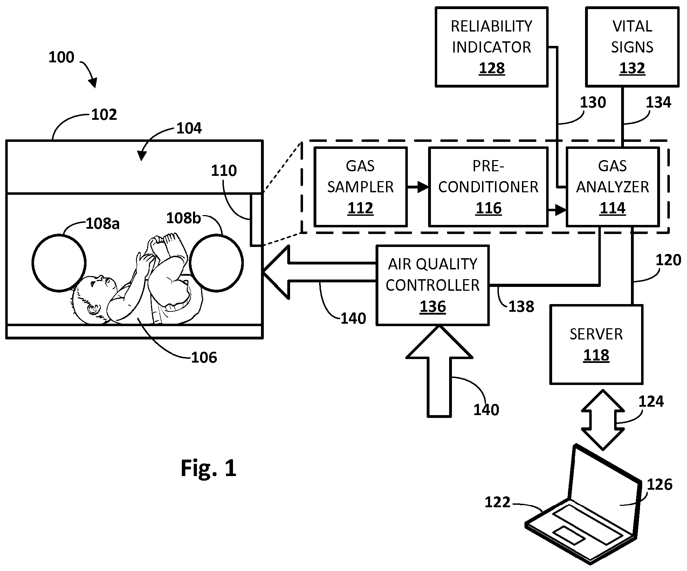

Referring to Fig. 1, in one embodiment, a neonatal incubator 100 (hereinafter, simply "incubator") may including a housing 102 that may be transparent, translucent, or even opaque. In various embodiments, incubator may define an interior 104 that may be a suitable environment for caring for an ill or premature newborn infant 106. While apparatus, systems, and methods described herein refer to "neonatal" incubators for humans, that is not meant to be limiting. Various techniques and apparatus described herein may be applied elsewhere, such as incubators for animals, or in other medically-protective environments such as negative pressure ventilators (e.g., "iron lungs").

In various embodiments, housing 102 may define various openings or ports that provide an interface between interior 104 and an exterior environment in which incubator 100 is placed *(e.g.,* in a neonatal intensive care unit, or "NICU"). For example, two hand-insertion portals, 108a and 108b, may be provided to permit medical personnel and/or a relative of infant 106 *(e.g.,* parents) to reach into interior 104 to perform various actions, such as taking medical measurements (e.g., blood pressure, blood samples), or simply to caress infant 106 with human touch. In some embodiments, portals 108a and 108b may be sealed with integral gloves (not depicted) to ensure that the person reaching into interior 104 of incubator 100 does not expose interior 104 to germs. In other embodiments, portals 108a and 108b may be selectively opened and closed, as needed, using various mechanisms.

In various embodiments, incubator 100 may be equipped with a neonatal gas analysis apparatus 110. In various embodiments, neonatal gas analysis apparatus 110 may include a gas sampler 112 configured to obtain one or more samples of gas from interior 104 of incubator 100. Gas sampler 112 may obtain samples of various sizes, depending on the circumstances, and may take multiple samples at various frequencies, also depending on the circumstances. In some embodiments, gas sampler 112 may include a pump (not depicted) and pump controller (also not depicted) that may be operated to control parameters of the pump's operation, such as the volume of gas sampled, the frequency at which gas is sampled, etc. In some embodiments, passive gas sampling may be employed, e.g., by placing a sorbent material in interior 104 for a predetermined amount of time.

In various embodiments, neonatal gas analysis apparatus 110 may also include a gas analyzer 114 that is in direct or indirect gaseous communication with gas sampler 112. In various embodiments, gas analyzer 114 may be configured to analyze one or more types of molecules contained in gas samples obtained by gas sampler 112, and to provide one or more signals of one or more health indicators based on the analysis. For example, in various embodiments, gas analyzer 114 may be configured to identify levels of one or more types of molecules contained in gas samples obtained by gas sampler 112.

A variety of types of molecules, such as biomolecules, carbohydrates, and/or volatile organic compounds ("VOCs") that might be found within an incubator may be analyzed by gas analyzer 114. These may include but are not limited to acetone, acetaldehyde, octadecane, nitrogen-containing VOCs such as acetamide or ammonia, sulfur-containing VOCs such as carbon disulfide, and/or ethers such as dimethyl ether or diethyl ether. Additionally or alternatively, straight chain alkenes may be analyzed, including methane, ethane, propane, butane, pentane, hexane, heptane, and/or octane. Additionally or alternatively, branched chain alkanes such as 2-methylpropane or methylbutane may be analyzed. In some embodiments, alkynes and/or non-cyclic alkenes such as propene or 1-Butene 2-Butene 1-Pentene may be analyzed. Additionally or alternatively, in various embodiments, one or more of the following may be analyzed by gas analyzer 114: benzyl and phenyl hydrocarbons, non-aromatic cyclic hydrocarbons, other hydrocarbons, alcohol volatiles, aldehyde volatiles, primary aliphatic acid volatiles, branched aliphatic acid volatiles, unsaturated acid volatiles, carboxylic acid volatiles, acetic acid esters, straight chain aliphatic acid esters, branched and cyclic aliphatic acetic acid esters, aromatic carboxylic acid esters, cyclic esters, other esters, non-cyclic alkenes (multiple double bonds), straight chain aliphatic ketones, branched chain and cyclic aliphatic ketones, di-ketones, unsaturated ketones, other ketones, and/or halogen containing VOCs. In some embodiments, gas analyzer 114 may identify when levels of bilirubin reach a threshold by measuring amounts of carbon monoxide production by infant 106, which at high enough levels can cause jaundice.

Gas analyzer 114 may be configured to provide, based on its analysis of gas samples obtained by gas sampler 112, a wide variety of health indicators pertaining to infant 106. In some embodiments, gas analyzer 114 may provide information about diseases such as bacterial infection. In some embodiments, gas analyzer 114 may provide information about VOCs found in exhaled breath, skin, urine and/or feces, which could be used to diagnose heath issues and/or to notify health personnel that infant 106 requires a change of diaper. In some embodiments, gas analyzer 114 may be configured to provide, based on its analysis, indicators of necrotizing enterocolitis ("NEC"), bronchopulmonary dysplasia ("BPD"), and/or pneumonia.

In some implementations, neonatal gas analysis apparatus 110 may include a preconditioner 116 in direct or indirect gaseous communication with gas sampler 112. In various embodiments, preconditioner 116 may be configured to "precondition" one or more gas samples obtained by gas sampler 112 to be in a state that is suitable for analysis by gas analyzer 114. For example, in various embodiments, preconditioner 116 may capture, isolate, and/or concentrate one or more types of molecules from one or more gas samples obtained by gas sampler 112.

In some embodiments, preconditioner 116 may include one or more surfaces *(e.g.,* beads) treated with a sorbent *(e.g.,* an adsorbent or absorbent). The sorbent may be configured, e.g., through absorption or adsorption, to capture and/or concentrate one or more types of molecules, such as one or more carbohydrates. In such embodiments, gas analyzer 114 may be configured to analyze the captured and concentrated one or more types of molecules.

In some embodiments, preconditioner 116 may include a temperature regulator (not depicted, may include, for instance, a heating element with a controller such as a thermostat) configured to alter a temperature of the one or more samples of gas obtained by gas sampler 112. The temperature regulator may be activated to increase and/or decrease a temperature of the gas samples collected by gas sampler 112, and/or to increase and/or decrease a temperature of captured and concentrated targeted types of molecules (e.g., in a sorbent material), in order to release the one or more targeted types of molecules.

In some embodiments, preconditioner 116 may be configured to concentrate gas sampled by gas sampler 112 by causing gas sampler 112 to sample for a predetermined time interval. In some embodiments, preconditioner 116 may cause gas samples captured by gas sampler 112 to be at a suitable flow and/or pressure, e.g., by compressing gas sampled by gas sampler 112 over such a predetermined time interval. Additionally or alternatively, in some embodiments, preconditioner 116 may be configured to remove various components from gas sampled by gas sampler 112. For example, in some embodiments, preconditioner 116 may remove water, *e.g.,* in the form of humidity and/or water vapor, from gas sampled by gas sampler 112.

In various embodiments, one or more components of neonatal gas analysis apparatus 110, such as gas analyzer 114, may be communicatively coupled with one or more computing systems, such as server 118 in Fig. 1, via a communication link 120. Various wired or wireless communication technologies may be used to implement communication link 120, including but not limited to The Institute of Electrical and Electronics Engineers ("IEEE") 802.3 standard (Ethernet), the IEEE 802.11 standard (Wi-Fi), Bluetooth, radio frequency identification ("RFID"), coded light, and so forth. In various embodiments, server 118 may be configured to provide, *e.g.,* to a remote computing device 122 over one or more communication links 124, data configured to enable remote computing device 122 to provide a user interface 126, as well as data indicative of the one or more signals of one or more health indicators provided by gas analyzer 114. User interface 126 may in turn provide output indicative of the one or more health indicators, such as charts, graphs, tables, numerical values, warnings (e.g., audible or visual), and so forth. Communication link 124 may be implemented using the same technology as communication link 120, and/or using a different technology.

In some embodiments, server 118 may be in communication with a plurality of neonatal gas analysis apparatus 110 installed on a plurality of incubators 100. In such a scenario, server 118 may be configured to provide health indicators and/or other data to remote computing devices *(e.g.,* 122) about multiple neonatal incubators, *e.g.,* across a NICU. In other embodiments, neonatal gas analysis apparatus 110 may communicate directly with one or more remote computing devices (e.g., 122), without an intermediate server. In some embodiments, neonatal gas analysis apparatus 110 may include an integral user interface, *e.g.,* a touch screen display mounted on housing 102, that enables a user to directly control and/or obtain heath indicators from neonatal gas analysis apparatus 110.

In some embodiments, a reliability indicator 128 may be in communication with one or more components of neonatal gas analysis apparatus 110, such as gas analyzer 114, over a communication link 130. Communication link 130 may be implemented using the same or different technologies as communications links 120 and/or 124. In some embodiments, reliability indicator 128 may be implemented as any combination of hardware and software, and could include, for instance, a field-programmable gate array ("FPGA") and/or an application-specific integrated circuit ("ASIC"). However reliability indicator 128 is implemented, it may be operably *(e.g.,* electrically) coupled with one or more sensors (not depicted) that detect when one or more openings of incubator 100 *(e.g.,* portals 108a and 108b) have been opened.

Based on this detection, reliability indicator 128 may be configured to provide, to gas analyzer 114, based on a state of isolation between interior 104 of incubator 100 and an exterior environment, a signal indicative of reliability of the one or more samples of gas obtained by gas sampler 112. For example, in some embodiments, reliability indicator 128 may provide gas analyzer 114 with a signal indicative of a passage of time since interior 104 of neonatal incubator 100 was exposed to the exterior environment. Gas analyzer 114 may be configured to calculate a measure of accuracy of the one or more signals of the one or more health indicators it provides based on the signal from reliability indicator 128. For example, gas analyzer 114 may calculate a measure of accuracy of the one or more signals of the one or more health indicators based on passage of time provided by reliability indicator 128. In some embodiments, if the passage of time since interior 104 was exposed to an outside environment fails to satisfy one or more thresholds, gas analyzer 114 may disregard and/or discard gas samples from gas sampler 112, and/or may wait until the passage of time since interior 104 was exposed to the outside environment satisfies the one or more thresholds before resuming analysis.

In some embodiments, a vital sign unit 132 may be in communication with one or more components of neonatal gas analysis apparatus 110, such as gas analyzer 114, via communication link 134. Communication link 134 may be implemented using the same or different technologies as previously-mentioned communication links *(e.g.,* 120, 124, 130). Vital sign unit 132 may be configured to obtain one or more vital signs, e.g., from infant 106, using various types of invasive, semi-invasive, and non-invasive probes, such as an electrocardiogram. Various vital signs may be obtained, including but not limited to oxygen saturation, signs pertaining to respiration, heart rate, blood pressure, glucose levels, and so forth. In various embodiments, vital sign unit 132 may provide, *e.g.,* to gas analyzer 114 over link 134, one or more signals indicative of these vital signs. Gas analyzer 114 may in turn incorporate these vital signs into its own analysis. For example, in some embodiments, gas analyzer 114 may determine that there likely is bacterial infection present based on a combination of increased heart rate, blood pressure, body temperature, and one or more biomarker findings. As another example, sepsis may be detected based on a combination of breath analysis and heart rate variability. In some embodiments, gas analyzer 114 may provide data indicative of the vital signs to server 118, so that they may be presented at user interface 126 alongside the other health indicators determined from analysis performed by gas analyzer 114.

In some embodiments, an air quality controller 136 may be in communication with one or more components of neonatal gas analysis apparatus 110, such as gas analyzer 114, via communication link 138. Communication link 138 may be implemented using the same or different technologies as previously-mentioned communication links *(e.g.,* 120, 124, 130, 134). Air quality controller 136 may be configured to receive, e.g., directly or indirectly from gas analyzer 114 and/or vital sign unit 132, data indicative of various health indicators, vital signs, and/or other conditions within incubator 100. In response to this received data, which air quality controller 136 may treat as feedback, air quality controller 136 may perform various air quality control measures, such as selectively diverting fresh air 140 into interior 104 of incubator 100. While not depicted in Fig. 1, in various embodiments, one or more filters, sorbent materials, or other air purification mechanisms may be deployed between air quality controller 136 and interior 104 to reduce and/or eliminate pollutants that might otherwise contaminate interior 104.

In addition to or instead of providing fresh air 140, in some embodiments, air quality controller 136 may monitor and/or control other attributes of the environment within interior 104 of incubator 100 based on data it receives from neonatal gas analysis apparatus 110, such as temperature, humidity, oxygen levels, and so forth. For example, gas analyzer 114 may provide data indicating that oxygen within interior 104 is too low. In response, air quality controller 136 may divert fresh air into interior 104 until gas analyzer 114 determines that oxygen levels within interior 104 are acceptable.

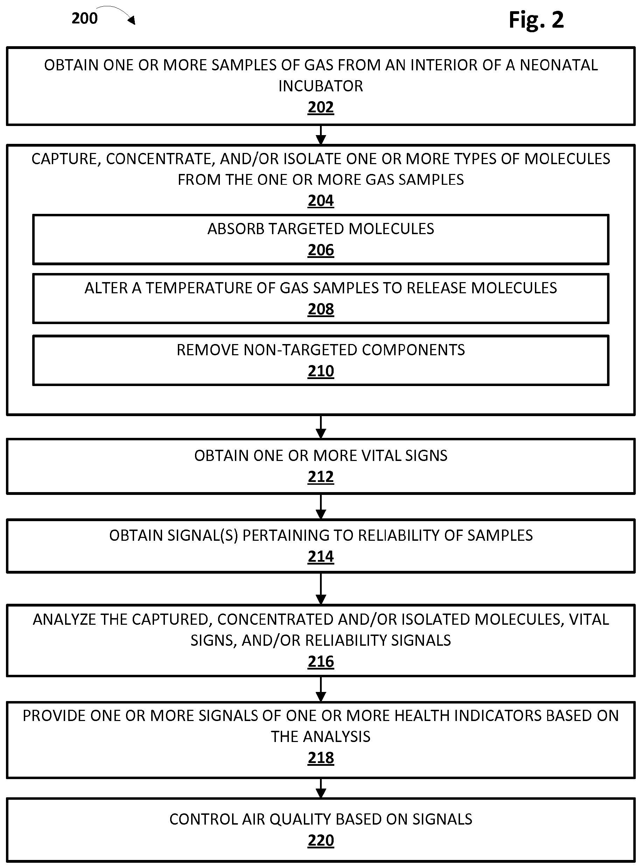

Referring now to Fig. 2, an example method 200 for monitoring a neonatal incubator (e.g., 100) using gas analysis is depicted. While the operations of method 200 are shown in a particular order, this is not meant to be limiting. One or more operations may be added, omitted, or reordered. At block 202, one or more samples of gas may be obtained from an interior *(e.g.,* 104) of an incubator *(e.g.,* 100), *e.g.,* by gas sampler 112. At block 204, the one or more gas samples obtained at block 202 may be preconditioned, e.g., by preconditioner 116, to capture, concentrate, and/or isolate one or more types of molecules (examples mentioned above) from the one or gas samples. For example, in some embodiments, and as indicated at block 206, one or more sorbent materials may be used to adsorb one or more targeted types of molecules. In some embodiments, at block 208, a temperature of the gas samples and/or sorbent materials may be altered, *e.g.,* to release one or more targeted types of molecules. In some embodiments, one or more non-targeted components, such as water, may be removed from the gas samples at block 210.

At block 212, one or more vital signs, such as heart rate, temperature, blood pressure, glucose levels, and so forth, may be obtained, e.g., by vital sign unit 132. At block 214, one or more signals pertaining to reliability of the gas samples may be obtained, e.g., from reliability indicator 128. At block 216, the molecules that were captured, concentrated, and/or isolated at block 204 may be analyzed, *e.g.,* by gas analyzer 114, *e.g.,* in combination with the vital signals obtained at block 212 and/or the reliability signals obtained at block 214.

At block 218, based on the analysis at block 216, gas analyzer 114 may provide one or more signals of one or more health indicators. For example, gas analyzer 114 may provide a signal indicative of high levels of one or more targeted types of molecules *(e.g.,* carborhydrates, VOCs), which may be indicative of various diseases or other medical conditions, or could even be indicative of presence of urine and/or feces within interior 104 of incubator. Additionally or alternatively, gas analyzer 114 may provide one or more signals that may be used as feedback, e.g., by air quality controller 136, to control air quality at block 220.

## Claims

1. A neonatal incubator (100), comprising:
- a housing (102) defining an interior (104) to house a neonate;
- a gas sampler (112) configured to obtain one or more samples of gas from the interior of the housing;
- a gas analyzer (114) in at least indirect gaseous communication with the gas sampler and configured to analyze one or more types of biomolecules in the one or more samples of gas that are carried in a neonate's breath, feces, skin and/or urine and provide one or more signals of one or more health indicators associated with the neonate, relating to diseases or health issues, based on the analysis,
wherein the gas analyzer is configured to provide data usable to render a user interface to provide output indicative of the one or more health indicators,
**characterized in that**
the biomolecules comprise one or more of:
acetone; acetaldehyde; octadecane; nitrogen-containing VOCs;sulfur-containing VOCs; ethers; straight chain alkenes;branched chain alkanes; alkynes and/or non-cyclic alkenes; benzyl and phenyl hydrocarbons; non-aromatic cyclic hydrocarbons; alcohol volatiles; aldehyde volatiles; primary aliphatic acid volatiles; branched aliphatic acid volatiles; unsaturated acid volatiles; carboxylic acid volatiles; acetic acid esters; straight chain aliphatic acid esters; branched and cyclic aliphatic acetic acid esters; aromatic carboxylic acid esters; cyclic esters; non-cyclic alkenes; straight chain aliphatic ketones; branched chain and cyclic aliphatic ketones; di-ketones; unsaturated ketones; halogen containing VOCs; and **further characterized in that** the neonatal incubator comprises
- a reliability indicator (128) operably coupled with the gas analyzer and configured to provide, to the gas analyzer, based on a state of isolation between the interior of the housing and an exterior environment, a signal indicative of reliability of the one or more samples of gas.

2. The neonatal incubator of claim 1, wherein the gas analyzer is further configured to calculate a measure of accuracy of the one or more signals of the one or more health indicators based on the signal from the reliability indicator.

3. The neonatal incubator of claim 1, wherein the reliability indicator is configured to provide the gas analyzer with a signal indicative of a passage of time since the interior of the housing was exposed to the exterior environment.

4. The neonatal incubator of claim 3, wherein the gas analyzer is further configured to calculate a measure of accuracy of the one or more signals of the one or more health indicators based on the passage of time.

5. The neonatal incubator of claim 1, wherein the reliability indicator is electrically coupled with one or more sensors that are configured to detect when one or more openings of the incubator have been opened.

6. The neonatal incubator of claim 1, further comprising a preconditioner (116) in gaseous communication with the gas sampler and gas analyzer and configured to capture, concentrate, or isolate the one or more types of biomolecules from the one or more samples of gas optionally using adsorption or temperature regulation.

7. The neonatal incubator of claim 6, wherein the preconditioner includes a sorbent material configured to capture and concentrate the one or more types of biomolecules.

8. The neonatal incubator of claim 7, wherein the preconditioner includes a temperature regulator configured to alter a temperature associated with the adsorbent material to release the one or more types of biomolecules from the sorbent material.

9. The neonatal incubator of claim 6, wherein the preconditioner includes a temperature regulator configured to alter a temperature of the one or more samples of gas to release the one or more types of biomolecules from the one or more samples of gas.

10. The neonatal incubator of claim 1, wherein the gas analyzer (114) is further configured to:
- receive, from a vital signs unit (132), one or more vital sign signals indicative of one or more vital signs of the neonate;
- analyze the one or more vital sign signals in combination with the one or more biomolecules and provide the one or more signals of one or more health indicators further based on the analysis of the one or more vital sign signals.

## Patentansprüche

1. Inkubator für Neugeborene (100), umfassend:
- ein Gehäuse (102), das einen Innenraum (104) zur Unterbringung eines Neugeborenen definiert;
- einen Gasprobenehmer (112), der dazu konfiguriert ist, eine oder mehrere Gasproben aus dem Inneren des Gehäuses zu entnehmen;
- einen Gasanalysator (114), der zumindest indirekt mit dem Gasprobennehmer in Gasverbindung steht und so konfiguriert ist, dass er eine oder mehrere Arten von Biomolekülen in der einen oder den mehreren Gasproben analysiert, die in der Atemluft, im Stuhl, in der Haut und/oder im Urin eines Neugeborenen enthalten sind und ein oder mehrere Signale eines oder mehrerer Gesundheitsindikatoren im Zusammenhang mit dem Neugeborenen bereitstellen, die sich auf Krankheiten oder Gesundheitsprobleme beziehen, basierend auf der Analyse,
wobei der Gasanalysator so konfiguriert ist, dass er Daten bereitstellt, die dazu verwendet werden können, eine Benutzerschnittstelle so zu gestalten, dass sie eine Ausgabe bereitstellt, die den einen oder die mehreren Gesundheitsindikatoren angibt,
**dadurch gekennzeichnet, dass** die Biomoleküle eines oder mehrere umfassen von:
Aceton; Acetaldehyd; Octadecan; stickstoffhaltige VOCs; schwefelhaltige VOCs; Ether; geradkettige Alkene; verzweigtkettige Alkane; Alkine und/oder nicht-zyklische Alkene; Benzyl- und Phenylkohlenwasserstoffe; nicht-aromatische zyklische Kohlenwasserstoffe; flüchtige Alkohole; flüchtige Aldehyde; flüchtige primäre aliphatische Säuren; flüchtige verzweigte aliphatische Säuren; flüchtige ungesättigte Säuren; flüchtige Carbonsäuren; Essigsäureester; geradkettige aliphatische Säureester; verzweigte und zyklische aliphatische Essigsäureester; aromatische Carbonsäureester; zyklische Ester; nicht-zyklische Alkene; geradkettige aliphatische Ketone; verzweigtkettige und zyklische aliphatische Ketone; Diketone; ungesättigte Ketone; halogenhaltige VOCs und weiter **dadurch gekennzeichnet, dass** der Inkubator für Neugeborene
- einen Zuverlässigkeitsindikator (128) umfasst, der betriebsmäßig mit dem Gasanalysator gekoppelt ist und dazu konfiguriert ist, dem Gasanalysator basierend auf einem Isolationszustand zwischen dem Inneren des Gehäuses und einer Außenumgebung ein Signal bereitzustellen, das die Zuverlässigkeit der einen oder mehreren Gasproben angibt.

2. Inkubator für Neugeborene nach Anspruch 1, wobei der Gasanalysator weiter dazu konfiguriert ist, ein Maß für die Genauigkeit des einen oder der mehreren Signale des einen oder der mehreren Gesundheitsindikatoren basierend auf dem Signal des Zuverlässigkeitsindikators zu berechnen.

3. Inkubator für Neugeborene nach Anspruch 1, wobei der Zuverlässigkeitsindikator so konfiguriert ist, dass er dem Gasanalysator ein Signal bereitstellt, das den Zeitablauf angibt, seitdem das Innere des Gehäuses der Außenumgebung ausgesetzt war.

4. Inkubator für Neugeborene nach Anspruch 3, wobei der Gasanalysator weiter dazu konfiguriert ist, ein Maß für die Genauigkeit des einen oder der mehreren Signale des einen oder der mehreren Gesundheitsindikatoren basierend auf dem Zeitablauf zu berechnen.

5. Inkubator für Neugeborene nach Anspruch 1, wobei der Zuverlässigkeitsindikator elektrisch mit einem oder mehreren Sensoren gekoppelt ist, die so konfiguriert sind, dass sie erkennen, wenn eine oder mehrere Öffnungen des Inkubators geöffnet wurden.

6. Inkubator für Neugeborene nach Anspruch 1, der weiter einen Vorkonditionierer (116) in Gasverbindung mit dem Gasprobenehmer und dem Gasanalysator umfasst und so konfiguriert ist, dass er die eine oder mehreren Arten von Biomolekülen aus der einen oder den mehreren Gasproben wahlweise unter Verwendung von Adsorption oder Temperaturregulierung auffängt, konzentriert oder isoliert.

7. Inkubator für Neugeborene nach Anspruch 6, wobei der Vorkonditionierer ein Sorptionsmaterial einschließt, das dazu konfiguriert ist, die eine oder mehreren Arten von Biomolekülen aufzufangen und zu konzentrieren.

8. Inkubator für Neugeborene nach Anspruch 7, wobei der Vorkonditionierer einen Temperaturregler einschließt, der dazu konfiguriert ist, eine dem Adsorptionsmaterial zugeordnete Temperatur zu ändern, um die eine oder mehreren Arten von Biomolekülen aus dem Sorptionsmaterial freizusetzen.

9. Inkubator für Neugeborene nach Anspruch 6, wobei der Vorkonditionierer einen Temperaturregler einschließt, der dazu konfiguriert ist, eine Temperatur der einen oder mehreren Gasproben zu ändern, um die eine oder mehreren Arten von Biomolekülen aus der einen oder mehreren Gasproben freizusetzen.

10. Inkubator für Neugeborene nach Anspruch 1, wobei der Gasanalysator (114) weiter dazu konfiguriert ist:
- von einer Vitalzeicheneinheit (132) ein oder mehrere Vitalzeichensignale zu empfangen, die ein oder mehrere Vitalzeichen des Neugeborenen angeben;
- Analysieren des einen oder der mehreren Vitalzeichensignale in Kombination mit dem einen oder den mehreren Biomolekülen und Bereitstellen des oder der mehreren Signale eines oder mehrerer Gesundheitsindikatoren basierend auf der Analyse des einen oder der mehreren Vitalzeichensignale.

## Revendications

1. Incubateur néonatal (100), comprenant :
- un boîtier (102) définissant un intérieur (104) pour loger un nouveau-né ;
- un échantillonneur de gaz (112) configuré pour obtenir un ou plusieurs échantillons de gaz de l'intérieur du boîtier ;
- un analyseur de gaz (114) en communication gazeuse au moins indirecte avec l'échantillonneur de gaz et configuré pour analyser un ou plusieurs types de biomolécules dans les un ou plusieurs échantillons de gaz qui sont transportés dans l'haleine, les selles, la peau et/ou l'urine d'un nouveau-né et fournir un ou plusieurs signaux d'un ou plusieurs indicateurs de santé associés au nouveau-né, relatifs à des maladies ou à des problèmes de santé, sur la base de l'analyse,
dans lequel l'analyseur de gaz est configuré pour fournir des données utilisables pour restituer une interface utilisateur afin de fournir une sortie indiquant les un ou plusieurs indicateurs de santé,
**caractérisé en ce que** les biomolécules comprennent un ou plusieurs parmi :
l'acétone ; l'acétaldéhyde ; l'octadécane ; les COV contenant de l'azote ; les COV contenant du soufre ; les éthers ; les alcènes à chaîne droite ; les alcanes à chaîne ramifiée ; les alcynes et/ou les alcènes non cycliques ; les hydrocarbures benzyliques et phényliques ; les hydrocarbures cycliques non aromatiques ; les composés volatils alcooliques ; les composés volatils aldéhydes ; les composés volatils acides aliphatiques primaires ; les composés volatils acides aliphatiques ramifiés ; les composés volatils acides insaturés ; les composés volatils acides carboxyliques ; les esters d'acide acétique ; les esters d'acide aliphatique à chaîne droite ; les esters d'acide acétique aliphatique ramifié et cyclique ; les esters d'acide carboxylique aromatiques ; les esters cycliques ; les alcènes non cycliques ; les cétones aliphatiques à chaîne droite ; les cétones aliphatiques à chaîne ramifiée et cycliques ; les di-cétones ; les cétones insaturées ; les COV contenant des halogènes et **caractérisé en outre en ce que** l'incubateur néonatal comprend
- un indicateur de fiabilité (128) couplé fonctionnellement à l'analyseur de gaz et configuré pour fournir, à l'analyseur de gaz, sur la base d'un état d'isolation entre l'intérieur du boîtier et un environnement extérieur, un signal indiquant la fiabilité des un ou plusieurs échantillons de gaz.

2. Incubateur néonatal selon la revendication 1, dans lequel l'analyseur de gaz est en outre configuré pour calculer une mesure de précision des un ou plusieurs signaux des un ou plusieurs indicateurs de santé sur la base du signal provenant de l'indicateur de fiabilité.

3. Incubateur néonatal selon la revendication 1, dans lequel l'indicateur de fiabilité est configuré pour fournir à l'analyseur de gaz un signal indiquant un temps écoulé depuis que l'intérieur du boîtier a été exposé à l'environnement extérieur.

4. Incubateur néonatal selon la revendication 3, dans lequel l'analyseur de gaz est en outre configuré pour calculer une mesure de précision des un ou plusieurs signaux des un ou plusieurs indicateurs de santé en fonction du temps écoulé.

5. Incubateur néonatal selon la revendication 1, dans lequel l'indicateur de fiabilité est couplé électriquement à un ou plusieurs capteurs qui sont configurés pour détecter lorsqu'une ou plusieurs ouvertures de l'incubateur ont été ouvertes.

6. Incubateur néonatal selon la revendication 1, comprenant en outre un préconditionneur (116) en communication gazeuse avec l'échantillonneur de gaz et l'analyseur de gaz et configuré pour capturer, concentrer ou isoler les un ou plusieurs types de biomolécules à partir des un ou plusieurs échantillons de gaz en utilisant éventuellement l'adsorption ou la régulation de température.

7. Incubateur néonatal selon la revendication 6, dans lequel le préconditionneur inclut un matériau sorbant configuré pour capturer et concentrer les un ou plusieurs types de biomolécules.

8. Incubateur néonatal selon la revendication 7, dans lequel le préconditionneur inclut un régulateur de température configuré pour modifier une température associée au matériau adsorbant afin de libérer les un ou plusieurs types de biomolécules du matériau sorbant.

9. Incubateur néonatal selon la revendication 6, dans lequel le préconditionneur inclut un régulateur de température configuré pour modifier une température des un ou plusieurs échantillons de gaz afin de libérer les un ou plusieurs types de biomolécules à partir des un ou plusieurs échantillons de gaz.

10. Incubateur néonatal selon la revendication 1, dans lequel l'analyseur de gaz (114) est en outre configuré pour :
- recevoir, d'une unité de signes vitaux (132), un ou plusieurs signaux de signes vitaux indiquant un ou plusieurs signes vitaux du nouveau-né ;
- analyser les un ou plusieurs signaux de signes vitaux en combinaison avec les une ou plusieurs biomolécules et fournir les un ou plusieurs signaux d'un ou plusieurs indicateurs de santé en outre sur la base de l'analyse des un ou plusieurs signaux de signes vitaux.
